# FASCICULE DE BREVET EUROPEEN

(11) **EP 1 146 818 B1**
(45) Date de publication et mention de la délivrance du brevet: **26.10.2005**
(21) Numéro de dépôt: 00901667.6
(22) Date de dépôt: 28.01.2000
(51) Int. Cl.: A61B 5/107

(54) **DISPOSITIF PORTATIF DE PRISE DE MESURES D'UNE DIMENSION PERIMETRIQUE CORPORELLE, NOTAMMENT DE LA JAMBE EN VUE DE LA PRESCRIPTION D'UNE ORTHESE DE CONTENTION**
MESSEINRICHTUNG FÜR KÖRPERUMFANG INSBESONDERE DAS BEIN IN BETRACHT ZUR VERSCHREIBUNG EINES KOMPRESSIONSSTRUMPFES
PORTABLE DEVICE FOR MEASURING A CORPORAL PERIMETRIC DIMENSION, I.E. THE LEG WITH A VIEW TO PRESCRIBING A COMPRESSION ORTHESIS

(30) Priorité: 28.01.1999 FR 9900961
(43) Date de publication de la demande: 24.10.2001
(73) Titulaire: Innothera Topic International (Societe Anonyme), 94110 Arcueil (FR)
(72) Inventeur: GARDON-MOLLARD, Christian, F-63400 Chamalières (FR)
(74) Mandataire: Dupuis-Latour, Dominique
(86) Numéro de dépôt international: PCT/FR2000/000200
(87) Numéro de publication internationale: WO 2000/044282

(56) Documents cités:
- WO-A-93/14728
- DE-A- 3 223 711
- US-A- 4 502 301

## Description

L'invention concerne un dispositif pour la prise de mesures d'une dimension périmétrique du corps d'un patient.

Cette invention est applicable de façon particulièrement avantageuse à la mesure des circonférences d'un membre, notamment de la jambe, à plusieurs niveaux de référence en vue de la prescription d'une orthèse compressive telle qu'un bas ou collant de contention.

L'invention n'est cependant pas limitée à cette application particulière, et s'applique aussi bien à la mesure d'autres membres, par exemple le bras pour la prescription, ici encore, d'une orthèse de contention, ou à d'autres mesures dimensionnelles telles que périmètre crânien, périmètre thoracique, tour de taille, tour de hanches, etc.

Jusqu'à présent, ces mensurations sont effectuées de façon manuelle au moyen d'un mètre-ruban ou d'un mètre enrouleur, la dimension à mesurer étant lue visuellement par l'opérateur d'après des graduations du ruban.

Les dimensions ainsi lues sont ensuite reportées par exemple sur une fiche de patient, et peuvent éventuellement servir, dans le cas de la prescription d'une orthèse de contention, au choix d'un modèle et/ou d'une taille particulière d'orthèse, à partir de tables de références éditées par les divers fabricants de ces articles.

Le DE-A-32 23 711 décrit un tel dispositif, comprenant un ruban à enrouleur pourvu d'une alternance de graduations claires et sombres permettant la mesure de la longueur déroulée par passage devant une cellule photoélectrique, et l'indication de la valeur correspondante sur un afficheur numérique.

Ce mode de prise de mesures n'est bien entendu pas à l'abri des erreurs de lecture, des effets de parallaxe, etc. au moment de la prise de mesures, ni des erreurs de report de la dimension lue.

Par ailleurs, dans le cas du choix d'une taille dans une gamme de dimensions, l'expérience de l'opérateur peut influer sur le choix dans le cas de morphologies particulières, ou de dimensions se trouvant à la frontière entre deux tailles successives, de sorte que des praticiens différents seront peut-être amenés à prescrire des modèles ou des tailles différents, donc un article qui n'est pas nécessairement celui qui est le mieux adapté au cas du patient.

On connaît, notamment d'après les US-A-4 181 959 et US-A-4 718 507, des dispositifs de mesure à mètre-ruban numériques qui comprennent en outre une commande de validation de ladite valeur numérique, actionnable par l'opérateur lorsque celui-ci a sorti la longueur de ruban correspondant à la mesure du périmètre recherché, ainsi que des moyens de mémorisation et de traitement de la valeur numérique une fois confirmée.

L'un des buts de la présente invention est de proposer un dispositif de prise de mesures automatique d'une ou plusieurs dimensions périmétriques du corps d'un patient qui permette de pallier ces diverses difficultés, en :
- supprimant les causes d'erreurs de lecture par l'opérateur,
- évitant tout report par ce dernier des dimensions lues, et
- offrant en outre une aide au choix de la taille et/ou du modèle le mieux adapté au patient considéré.

Le dispositif de l'invention est du type connu d'après le DE-A-32 23 711 précité, c'est-à-dire comprenantles caractéristiques énoncées au préambule de la revendication 1. Selon l'invention, il est en outre pourvu des caractéristiques énoncées à la partie caractérisante de la revendication 1. Les sous-revendications visent des formes particulières de mise en oeuvre.

On va maintenant décrire un exemple de réalisation de l'invention, en référence aux dessins annexés.
La figure 1 est une vue d'ensemble d'un premier mode de réalisation du dispositif de prise de mesure selon l'invention.
La figure 2 illustre un autre exemple de réalisation de ce dispositif, dépourvu de sa coque externe pour montrer les différents éléments qui le composent.

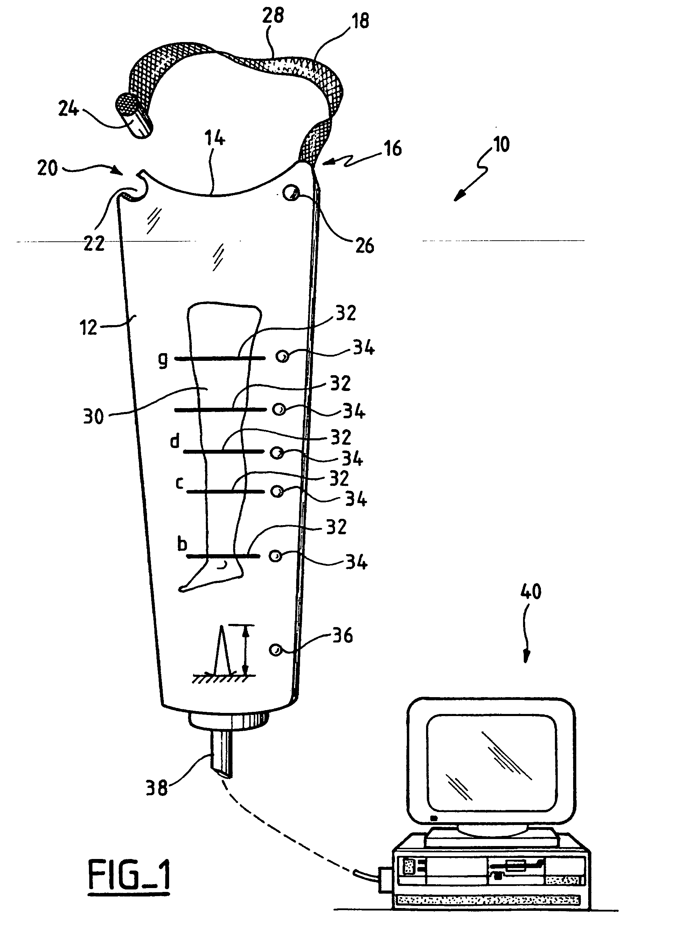

La figura 1 illustre une première forme de réalisation du dispositif de l'invention. Sur cette figure, la référence 10 désigne de façon générale le dispositif de l'invention, qui se présente avantageusement sous la forme d'un appareil portable, de faible encombrement, susceptible d'être tenu à la main, avec par exemple des dimensions du même ordre que celle d'une télécommande de téléviseur.

Ce dispositif 10 comporte un boîtier 12 dont la forme est conçue pour qu'il soit aisément tenu dans une main, avec à l'une de ses extrémités une partie concave 14 destinée à être appliquée contre la jambe à mesurer (dans le cas d'une application à la mesure des dimensions d'une jambe en vue de la prescription d'une orthèse de contention).

À l'une des extrémités de la partie concave 14 se trouve en 16 une fente de laquelle sort un ruban (ou fil) 18 monté sur un enrouleur automatique.

A l'extrémité opposée 20 est prévu un moyen de retenue, par exemple une encoche 22, susceptible de recevoir une pièce 24, par exemple cylindrique, fixée à l'extrémité libre du ruban 18.

Le ruban peut être extrait sur la longueur voulue en le déroulant d'un tambour récepteur (non représenté) du système enrouleur, ce tambour comportant un système anti-retour susceptible d'être libéré par un bouton 26 de rappel du ruban, avec des moyens mécaniques appropriés et connus.

Pour effectuer la mesure, l'opérateur déroule le ruban 18, passe celui-ci autour du membre et vient loger le cylindre 24 dans l'encoche 22. Il appuie alors sur le bouton de rappel 26 de manière à tendre le ruban 18 afin que celui-ci épouse au plus près le membre, qui va donc se trouver entouré par le ruban 18 et la partie concave 14.

Pour mesurer le périmètre ainsi déterminé, le dispositif comporte des moyens codeurs appropriés, en eux-mêmes connus, par exemple un codeur de rotation du tambour enrouleur du ruban 18, ou encore un codeur optique ou magnétique coopérant avec des graduations 28 dessinées sur le ruban, le codeur comptant alors le nombre de graduations défilant devant lui et déterminant ainsi la longueur de ruban enroulée.

Ce codeur, dont la précision souhaitable est de l'ordre du millimètre, peut être de type incrémental ou absolu, dès lors qu'il permet de déterminer sans ambiguïté la longueur de ruban déroulée et donc, en tenant compte de la longueur de la partie concave 14, la valeur de la circonférence que l'on mesure.

On peut notamment utiliser à cet effet un ruban transparent, par exemple en polycarbonate de 175 µm d'épaisseur, muni de stries de 1 mm de largeur alternativement noires et blanches sérigraphiées sur le ruban. La sérigraphie du ruban comprend avantageusement non seulement les stries nécessaires au capteur optique, mais également une graduation métrique pour contrôle visuel. Les stries peuvent être détectées par transparence à l'aide d'un couple émetteur/récepteur infrarouge, par exemple un couple émetteur/récepteur Honeywell SEP8506/HLC2705, procurant une précision typique de 1 mm sur une étendue de mesure maximale de 1000 mm.

Le boîtier 12 comporte également une représentation 30 d'une jambe, avec des indications donnant les différents niveaux de mesures (cheville, mollet, genou, bas de cuisse, haut de cuisse) et, en regard de chaque niveau 32, un bouton-poussoir correspondant.

On prévoit également un bouton-poussoir additionnel 36 correspondant à la mesure de la hauteur d'entrejambe.

Cette hauteur d'entrejambe peut être mesurée de la même façon que les périmètres de la jambe, c'est à dire au moyen du ruban 18. Néanmoins, dans une variante avantageuse, cette hauteur est mesurée par des moyens spécifiques, tels qu'un système télémétrique à ultrasons réalisant une mesure du temps de propagation aller-retour d'un signal ultrasonore entre le dispositif 10 et le sol. Le dispositif télémétrique peut être par exemple un ensemble émetteur/récepteur Murata MA40B8S/MA40B8R émettant des trains d'ondes à 44 kHz et procurant une précision de l'ordre de 5 mm sur une plage de mesure allant jusqu'à 1000 mm. Un niveau à bulle placé sur l'appareil permet éventuellement à l'opérateur de positionner correctement celui-ci pour assurer une bonne assiette garantissant la validité de la mesure de hauteur.

Le boîtier 12 étant relié par une liaison 38 à un micro-ordinateur 40, l'appui sur chacun des boutons 34 constituera une validation de la mesure effectuée avec le ruban 18, autorisant la transmission au logiciel du micro-ordinateur 40 de la valeur numérique délivrée par le codeur, ainsi que d'un code indiquant de quelle mesure il s'agit (niveau ou hauteur d'entrejambe).

Une fois les différentes mesures effectuées et validées par appui sur les boutons 34 et 36, les données correspondantes sont mémorisées et traitées par le logiciel du micro-ordinateur 40.

L'interfaçage entre le boîtier 12 et le micro-ordinateur 40 peut être réalisé de diverses manières.

Tout d'abord, la liaison entre le boîtier et le micro-ordinateur peut être une liaison par fil, au moyen d'un câble souple et suffisamment long pour une prise de mesures facile, ou bien une transmission sans fil, par exemple par infrarouge.

Par ailleurs, la transmission des données entre boîtier et micro-ordinateur peut se faire soit en direct, soit en différé. Dans ce dernier cas, le boîtier est un boîtier autonome qui permet de prendre les mesures de façon indépendante du micro-ordinateur. Les différentes valeurs sont stockées dans une mémoire de données incorporées au boîtier, et ces données sont transmises ultérieurement au micro-ordinateur, par exemple par transmission infrarouge, ou en posant le boîtier sur une base reliée au micro-ordinateur. Cette configuration permet d'effectuer la prise de mesure dans un endroit isolé, qui peut être éloigné du micro-ordinateur ; une fois ces données saisies et mémorisées dans le boîtier, l'opérateur rejoint le micro-ordinateur pour y transférer les données qu'il vient de saisir et effectuer les divers traitements que l'on va décrire ci-dessous.

Il est également possible de prévoir une version plus élaborée du boîtier, dans laquelle celui-ci comporte un processeur effectuant en interne le traitement des données saisies, ainsi qu'un afficheur à cristaux liquides permettant de donner immédiatement à l'opérateur les résultats du traitement. La liaison avec le micro-ordinateur n'est réalisée par exemple qu'en fin de journée, afin de permettre la mise à jour du fichier des patients à partir des diverses mensurations et prescriptions qui auront été effectuées dans le courant de la journée.

Le traitement opéré par le logiciel consiste à indiquer, à partir des diverses valeurs relevées, la taille de bas ou collant de contention la mieux adaptée à la morphologie du patient.

Cette sélection est effectuée à partir de tables mémorisées par le micro-ordinateur, correspondant aux différentes tailles des différents modèles d'un même fabricant, ou de plusieurs fabricants.

Le logiciel peut proposer à l'opérateur une référence (fabricant, modèle et taille) unique, ou une référence chez plusieurs fabricants, ou plusieurs références chez un ou plusieurs fabricants, par exemple dans le cas où la morphologie de la jambe du patient se situe à la limite entre deux tailles.

Si le logiciel ne trouve pas dans les tables de référence satisfaisante, il pourra alors indiquer qu'il est nécessaire de passer à un modèle sur mesure, pour lequel il sera éventuellement possible de configurer le logiciel afin de prendre les différentes dimensions nécessaires à cet effet.

Les paramètres de choix du modèle peuvent également, outre les mensurations, prendre en compte la pression de contention souhaitée, en particulier lorsque c'est le médecin phlébologue qui effectue lui-même les relevés. Le logiciel pourra ainsi proposer la meilleure référence, ou les meilleures références, compte tenu à la fois de la morphologie et de la pathologie du patient. Il pourrait ainsi estimer les pressions exercées sur la jambe pour une classe de contention donnée, en fonction des mensurations, par exemple lorsque l'on pourrait prescrire une classe II pour obtenir la pression d'une classe I sur une anatomie atypique.

Enfin, le logiciel pourra mettre à jour un fichier des patients et afficher et/ou éditer une fiche de patient récapitulant les mensurations, les références de bas ou collants proposés ou sélectionnés, etc.

On comprendra que le logiciel de traitement peut être très aisément et régulièrement mis à jour avec des données pratiques sur la contention : avantages de certains modèles pour certaines anatomies, valeur des pressions, nouvelle gamme de couleurs ou nouveaux modèles, aide pour du sur-mesure, etc.

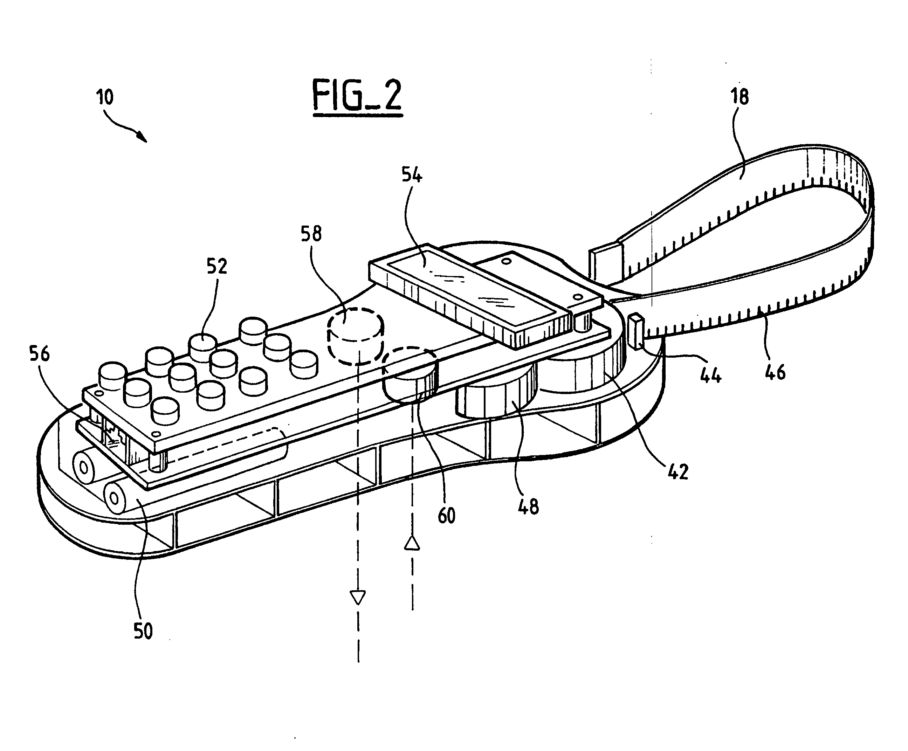

La figure 2 montre une autre réalisation du dispositif 10, où notamment le ruban 18 est un ruban interchangeable enfermé dans une cartouche amovible 42 : ceci permet de changer le ruban aussi souvent que de besoin, ou même de prévoir un ruban jetable, renouvelé à chaque nouvelle utilisation. A la sortie de la cartouche 42, le ruban passe devant un capteur optique 44 assurant le comptage des stries 46 imprimées sur le ruban. Un bouton-poussoir latéral 48 permet de commander le retour automatique du ruban 18 dans sa cartouche 42.

Le dispositif 10 est un dispositif autonome alimenté par des piles 50, avec un clavier 52 pour l'introduction des différentes commandes et la validation des mesures, et un afficheur à cristaux liquides 54 permettant de présenter des messages à l'utilisateur lui indiquant les opérations à réaliser.

L'appareil est pourvu en outre d'un connecteur 56 permettant une liaison, par exemple en fin de journée, avec un micro-ordinateur distant pour la récupération des données et la mise à jour des informations. Le dispositif comporte également un ensemble émetteur 58/récepteur 60 pour la mesure de hauteur, en particulier pour la détermination de la hauteur d'entrejambe.

L'utilisation d'un ensemble clavier/afficheur permet d'augmenter les fonctions disponibles et de simplifier l'utilisation, tout particulièrement dans le cas d'un appareil autonome.

On peut ainsi prévoir d'instaurer un dialogue entre l'appareil et l'utilisateur.

Tout d'abord, l'utilisateur effectue les différentes mesures, en validant chacune d'entre elles par pression sur les touches correspondant au point de mesure considéré ; le numéro de chaque mesure validée s'affiche sur l'écran. L'ordre de validation des mesures est indifférent, et chaque mesure peut être validée plusieurs fois. Le bouclage ne s'interrompt que lorsque tous les points de mesure ont été validés (à tout moment l'utilisateur peut annuler les mesures et revenir au début du cycle en pressant une touche d'annulation).

Lorsque la mesure est terminée, le dispositif détermine et affiche la taille du bas adapté aux mesures.

L'opérateur effectue alors la mesure de hauteur d'entrejambe, ce qui permet de déterminer le paramètre de hauteur.

Enfin, l'opérateur saisit le modèle de bas, le dispositif déterminant et affichant alors la référence correspondante. La sauvegarde de la mesure en mémoire interne est alors proposée à l'utilisateur et, s'il accepte, il est invité à saisir le nom du client. Le nom, les valeurs mesurées et la référence du bas sont alors sauvegardés en mémoire interne et ajoutés à la base de données clients.

## Revendications

1. Un dispositif portatif de prise de mesures d'une dimension périmétrique du corps d'un patient, notamment pour la mesure de circonférences de la jambe à plusieurs niveaux de référence en vue de la prescription d'une orthèse de contention, ce dispositif comprenant un ruban (18) ou fil souple monté sur un système enrouleur, des moyens de détermination de la longueur de ruban sortie de l'enrouleur comportant un codeur transformant un paramètre mécanique représentatif de la longueur de ruban déroulé en un signal électrique, et des moyens de conversion recevant ce signal électrique et délivrant en sortie une valeur numérique correspondante,
**caractérisé en ce qu'**il est prévu :
- au moins une commande de validation (34, 36) de ladite valeur numérique, actionnable par l'opérateur lorsque celui-ci a sorti la longueur de ruban correspondant à la mesure du périmètre recherché, et
- des moyens de mémorisation et de traitement de la valeur numérique une fois validée, ces moyens comprenant des moyens de calcul automatique d'une taille d'orthèse à prescrire dont des moyens de mémorisation d'une table d'au moins une gamme de tailles d'un modèle d'orthèse, et des moyens de sélection automatique, dans cette table, d'au moins un couple modèle/taille optimal en fonction des mesures relevées et mémorisées .

2. Le dispositif de la revendication 1, dans lequel le ruban est logé dans une cartouche interchangeable.

3. Le dispositif de la revendication 1, comprenant en outre des moyens spécifiques de mesure, de numérisation et de mémorisation d'une donnée de hauteur, notamment de hauteur d'entrejambe.

4. Le dispositif de la revendication 1, comportant une pluralité de commandes de validation (34, 36) distinctes, correspondant à différentes mesures successives prédéfinies.

5. Le dispositif de la revendication 1, comportant une face concave (14) appliquée contre la région du corps à mesurer, l'une des extrémités (16) de cette face concave comportant un orifice de sortie du ruban, formant origine de mesure des longueurs, et l'extrémité opposée (20) comportant des moyens (22) de maintien de l'extrémité libre (24) du ruban.

## Patentansprüche

1. Tragbare Vorrichtung zur Vomahme von Messungen einer Umfangsdimension des Körpers eines Patienten, insbesondere zur Messung von Umfängen des Beins auf verschiedenen Referenz-Höhen, im Hinblick auf die Verschreibung eines Kompressionstrumpfes, wobei diese Vorrichtung ein geschmeidiges Band (18) oder Faden auf einem Aufroll-System, Mittel zur Bestimmung der Länge des Bandes, das aus dem Aufroll-System herausgekommen ist, die einen Kodierer aufweisen, welcher einen mechanischen Parameter, der repräsentativ für die Länge des entrollten Bandes ist, in ein elektrisches Signal transformiert, und Umwandlungsmittel umfasst, welche dieses elektrische Signal empfangen und am Ausgang einen entsprechenden digitalen Wert liefern,
**dadurch gekennzeichnet, dass** vorgesehen ist:
- mindestens eine Steuerung zur Validierung (34, 36) des digitalen Werts, betätigbar durch den Bediener wenn dieser das Band in der Länge herausgezogen hat, welche dem gesuchten Maß des Umfangs entspricht, und
- Mittel zur Speicherung und zur Verarbeitung des einmal validierten numerischen Werts, wobei diese Mittel Mittel zur automatischen Berechnung einer Größe einer zu verschreibenden Orthese umfassen, sowie Mittel zur Speicherung einer Tabelle von mindestens einem Bereich von Größen eines Orthesenmodells, und Mittel zur automatischen Auswahl, aus dieser Tabelle, von mindestens einem optimalen Modell/Größen-Paar, abhängig von den erhobenen und gespeicherten Messungen.

2. Vorrichtung gemäß Anspruch 1, in welcher das Band in einer austauschbaren Kartusche enthalten ist.

3. Vorrichtung gemäß Anspruch 1, ferner spezifische Meßmittel enthaltend, zur Digitalisierung und Speicherung eines Höhen-Datums, insbesondere der Höhe des Schritts.

4. Vorrichtung gemäß Anspruch 1, eine Vielzahl von unterschiedlichen Validationssteuerungen (34, 36) enthaltend, verschiedenen vordefinierten aufeinander folgenden Messungen entsprechend.

5. Vorrichtung gemäß Anspruch 1, eine konkave Seite (14) aufweisend, welche gegen den zu messenden Bereich des Körpers angelegt wird, wobei eines der Enden (16) dieser konkaven Seite eine Ausgangsöffnung des Bandes aufweist, welche den Ursprung für die Messung von Längen bildet, und wobei das gegenüber liegende Ende (20) Mittel (22) zum Halten des freien Endes (24) des Bandes aufweist.

## Claims

1. A portable device for measuring a perimeter dimension of a patient's body, in particular for measuring circumferences of the leg at various reference levels in order to prescribe a compression orthosis, the device comprising a flexible tape (18) or wire mounted on a winding system, means for determining the length of tape wound out from the winder including an encoder for transforming a mechanical parameter representative of the length of wound-out tape into an electrical signal, and converter means receiving said electrical signal and outputting a corresponding digital value,
the device being **characterized in that** there are provided:
- at least one validation control (34, 36) for validating said digital value and actuatable by the operator once the operator has extracted a length of tape that corresponds to the desired perimeter measurement; and
- storage and processing means for storing and processing the digital value once validated, said means including means for automatically calculating a size of orthosis to be prescribed, including means for storing a table of at least one range of sizes of a model of orthosis, and automatic selection means for selecting at least one model-and-size pair from said table, which pair is optimal as a function of the measurements as taken and stored.

2. The device of claim 1, in which the tape is housed in an interchangeable cartridge.

3. The device of claim 1, further comprising specific means for measuring, digitizing, and storing height data, in particular inside leg length data.

4. The device of claim 1, comprising a plurality of distinct validation controls (34, 36) corresponding to different predefined successive measurements.

5. The device of claim 1, including a concave face (14) for applying against the region of the body to be measured, one of the ends (16) of said concave face having a tape outlet orifice forming the origin for length measurements, and the opposite end (20) having means (22) for holding the free end (22) of the tape.
